## Europäisches Patentamt

## European Patent Office

## Office européen des brevets

(19)

(11) Veröffentlichungsnummer: **0 178 728**
**B1**

(12) **EUROPÄISCHE PATENTSCHRIFT**

(45) Veröffentlichungstag der Patentschrift:
**10.05.89**

(21) Anmeldenummer: **85201648.4**

(22) Anmeldetag: **09.10.85**

(51) Int. Cl.⁴: **A 61 B 6/06,** A 61 B 6/00

(54) **Blendenanordnung für ein Mammographie-Röntgengerät.**

(30) Priorität: **13.10.84 DE 3437576**

(43) Veröffentlichungstag der Anmeldung:
**23.04.86 Patentblatt 86/17**

(45) Bekanntmachung des Hinweises auf die Patenterteilung:
**10.05.89 Patentblatt 89/19**

(84) Benannte Vertragsstaaten:
**DE FR GB**

(56) Entgegenhaltungen:
**DE-A-2 019 391**
**US-A-2 570 820**
**US-A-3 163 762**
**US-A-4 097 748**

(73) Patentinhaber: **Philips Patentverwaltung GmbH, Wendenstrasse 35 Postfach 10 51 49, D-2000 Hamburg 1 (DE)**
(84) Benannte Vertragsstaaten: **DE**

(73) Patentinhaber: **N.V. Philips' Gloeilampenfabrieken, Groenewoudseweg 1, NL- 5621 BA Eindhoven (NL)**
(84) Benannte Vertragsstaaten: **FR GB**

(72) Erfinder: **Bauer, Manfred, Heinrich Strasse 8A, D-2077 Brunsbek (DE)**

(74) Vertreter: **Hartmann, Heinrich, Dipl.- Ing., Philips Patentverwaltung GmbH Wendenstrasse 35 Postfach 10 51 49, D-2000 Hamburg 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

## Beschreibung

Die Erfindung betrifft eine Blendenanordnung für ein Mammographie-Röntgengerät zur Ausblendung eines Strahlenfeldes einer Strahlenquelle mit einem mit der Strahlenquelle verbundenen Tubus, der das Strahlenbündel entsprechend den äußeren Abmessungen eines Röntgenbildaufnehmers begrenzt, und einer darin befindlichen Blende, die das Strahlenbündel weiter eingrenzt. Ein Mammographie-Röntgengerät dieser Gattung ist aus US-A-4 097 748 bekannt.

Eine derartige Blendenanordnung befindet sich für Zwecke der sogenannten Vergrößerungsmammographie im praktischen Einsatz. Die Blende, die das Strahlenbündel noch weiter eingrenzen soll, besteht dabei aus einer auswechselbaren Platte, die in einer zum Bildaufnehmer parallelen Ebene in den Tubus eingeschoben ist. Die Platte ist an ihrer einen Seite mit einer abgerundeten Ausnehmung versehen, so daß ein entsprechendes Strahlenfeld im Bereich des vorderen Randes des Bildaufnehmers (der vordere Rand ist der bei einer Mammaaufnahme der Brustwand der Patientin zugewandte Rand des Bildaufnehmers) ausgeblendet wird. Außerdem enthält die Platte noch mindestens eine Öffnung, durch die im Bereich des hinteren Randes des Bildaufnehmers ein Strahlenfeld ausgeblendet wird, mit Hilfe dessen Patientendaten oder dergleichen auf den Bildaufnehmer aufbelichtet werden können.

Größe und Form des ausgeblendeten Strahlenfeldes sind dabei von der Größe und der Form der Ausnehmung in der Blendenplatte abhängig. Zur Ausblendung eines anderen Strahlenfeldes muß die Blendenplatte daher gegen eine Blendenplatte mit entsprechend geformter Ausnehmung ausgetauscht werden.

Aufgabe der vorliegenden Erfindung ist es, eine Blendenanordnung der eingangs genannten Art so auszugestalten, daß eine Veränderung des Strahlenfeldes ohne Wechsel der Blende möglich ist.

Diese Aufgabe wird erfindungsgemäß dadurch gelöst, daß die Blende durch eine Blendenklappe aus einem die Röntgenstrahlung absorbierenden Material gebildet wird, die im Tubus um eine zum vorderen Rand des Bildaufnehmers parallele Achse schwenkbar angeordnet ist.

Je nach Schwenkstellung der Blendenklappe blendet diese ein mehr oder weniger großes Strahlenfeld aus, so daß das Strahlenfeld durch Schwenken der Blendenklappe den jeweiligen Erfordernissen angepaßt werden kann.

Eine bevorzugte Weiterbildung der Erfindung sieht vor, daß die hintere Ecke der Kante der Blendenklappe mit der Schwenkachse zusammenfällt und daß die Schwenkachse im Strahlenbündel angeordnet ist. Die mit der Schwenkachse zusammenfallende hintere Ecke der der Strahlenquelle zugewandten Kante der Blendenklappe bildet die Begrenzung eines weiteren Strahlenfeldes im Bereich des hinteren Randes des Bildaufnehmers, das zu Aufbelichtungszwecken verwendet werden kann. Die Größe dieses Strahlenfeldes ist unabhängig von der Schwenkstellung der Blendenklappe und somit unabhängig von der Größe des für die eigentliche Mammaaufnahme benötigten Strahlenfeldes.

Die Erfindung wird nachstehend anhand der Zeichnungen näher erläutert. Es zeigen:

Fig. 1      ein Ausführungsbeispiel eines erfindungsgemäßen Röntgen-Mammographiegerätes,

Fig. 2 und 3      verschiedene Halbschnitte durch den Tubus und die Blendenklappe eines derartigen Gerätes und

Fig. 4 bis 6      verschiedene Ausführungsformen der Blendenklappe und die dadurch auf dem Bildaufnehmer hervorgerufenen Strahlenfelder.

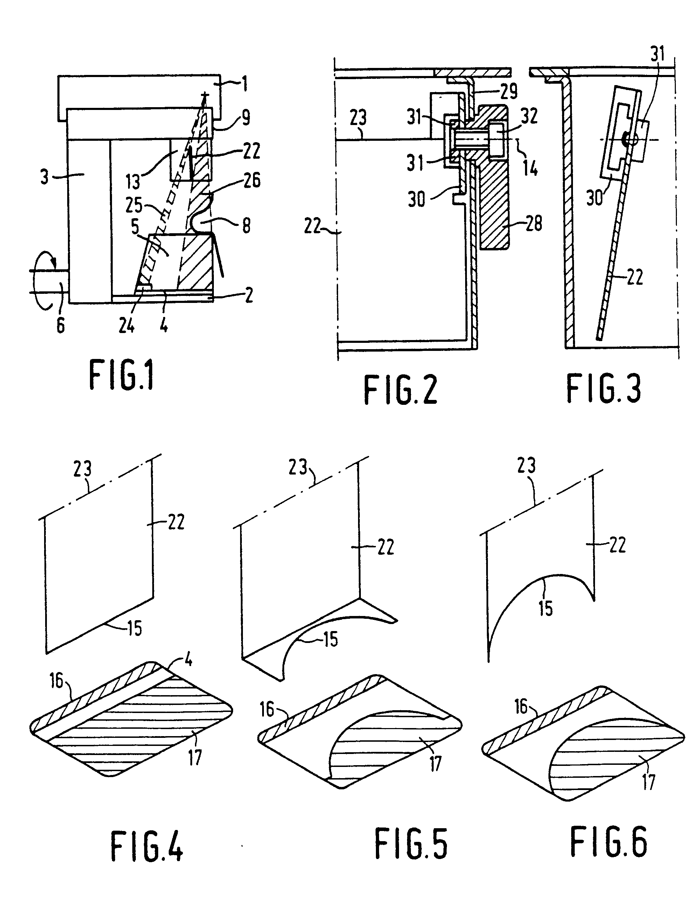

Das in Fig. 1 dargestellte Röntgen-Mammographiegerät umfaßt einen Röntgenstrahler 1 - im allgemeinen in Form einer in einem Schutzgehäuse befindlichen Röntgenröhre - und einen Aufnahmetisch 2, die über einen Träger 3 miteinander verbunden sind. Auf dem Aufnahmetisch befindet sich ein Bildaufnehmer 4, z. B. ein Film oder eine Filmkassette, sowie ein Vergrößerungstisch 5 aus strahlentransparentem Material, auf dem die Mamma 8 einer zu untersuchenden Patientin gelagert wird. Durch die Verwendung des Vergrößerungstisches befinden sich die aufzunehmende Mamma 8 und der Bildaufnehmer 4 in einigem Abstand voneinander, so daß bei einer Mammographie die Mamma 8 vergrößert auf dem Bildaufnehmer 4 abgebildet wird.

Der Träger 3 ist um eine Achse 6 drehbar, so daß auch Mammaaufnahmen mit horizontal verlaufendem Strahlengang möglich sind.

Der Röntgenstrahler 1 ist über einen Halter 9 mit dem Träger 3 verbunden. An dem Halter ist ein Tubus 13 befestigt, dessen unterer Rand das von der Strahlenquelle emittierte Strahlenbündel entsprechend den äußeren Abmessungen des (rechteckigen) Bildaufnehmers begrenzt. Innerhalb des Tubus 13 ist eine Blendenklappe 22 aus einem die Röntgenstrahlung absorbierenden Material angeordnet.

Die Blendenklappe 22 ist um eine zum vorderen Rand des Bildaufnehmers 4 parallele, d.h. zur Zeichenebene der Fig. 1 senkrechte Achse schwenkbar, die mit der der Strahlenquelle 1 zugewandten (oberen) hinteren Kantenecke der Blendenklappe 22 zusammenfällt.

Durch die erwähnte obere hintere Kantenecke der Blendenklappe 22 und durch die Unterkante der hinteren (linken) Seitenwand des Tubus 13 wird ein Strahlenbündel 25 ausgeblendet, das durch ein unmittelbar vor dem Bildaufnehmer angeordnetes Aufbelichtungsschild 24 hindurch ein streifenförmiges Feld am hinteren Rand des Bildaufnehmers 4 belichtet.

Der untere Rand der Blendenklappe 22 und des Tubus 13 begrenzen ein zweites Strahlenbündel 26, das durch die Mamma hindurchtritt und den Bildaufnehmer im Bereich seines vorderen (brustwandnahen) Bereiches belichtet. Wenn die Blendenklappe um ihre Schwenkachse im Uhrzeigersinn geschwenkt wird, nimmt der Querschnitt des Strahlenbündels 26 bis auf einen Maximalwert zu, wobei die Blendenklappe 22 und der Brennfleck der Strahlenquelle 1 in einer Ebene liegen. Bei einer Schwenkung der Blendenklappe im Gegenuhrzeigersinn nimmt der Querschnitt des Strahlenbündels ab. Da die Kantenecke der Blendenklappe, die die Abmessungen des Strahlenbündels 25 definiert, mit der Schwenkachse zusammenfällt, ändert das Strahlenbündel 25 seinen Querschnitt bei einer Schwenkung der Blendenklappe 22 nicht.

Die optimale Stellung der Blendenklappe 22 wird in an sich bekannter Weise mittels eines aus einer Lichtquelle und einem röntgenstrahlendurchlässigen Spiegel bestehenden nicht näher dargestellten Lichtvisiers bestimmt, das im Halter 9 untergebracht sein kann und durch das ein Lichtbündel auf den Bildaufnehmer 4 projiziert wird, das sich mit dem Röntgenstrahlenbündel deckt.

Die erfindungsgemäße Blendenanordnung ist auch anwendbar, wenn bei der Mammographie kein Vergrößerungstisch 5 benutzt wird und die Mamma unmittelbar über der Bildaufnahmevorrichtung plaziert wird, so daß sich praktisch eine nahezu größengetreue Projektion der Mamma auf den Film ergibt. Da somit ein größerer Teil des Formats des Bildaufnehmers 4 für die eigentliche Mammaaufnahme nicht benötigt wird, ist hier die Plazierung von Aufbelichtungsschildern einfacher, so daß das Strahlenbündel 25 unter Umständen nicht erforderlich ist. Die Schwenkachse der Blendenklappe 22 kann im Tubus 13 dann außerhalb des durch dessen Unterkanten definierten Strahlenganges angeordnet werden, so daß mit Hilfe der Blendenklappe 22 und des Tubus 13 nur ein Strahlenfeld definiert wird.

Fig. 2 zeigt einen Querschnitt durch eine Hälfte des Tubus und der Blendenklappe in einer zur Zeichenebene der Fig. 1 senkrechten Ebene, während Fig. 3 einen solchen Querschnitt in der Zeichenebene darstellt. Wie aus Fig. 2 ersichtlich ist, besitzt die Blendenklappe 22 an ihrem seitlichen Rand jeweils einen senkrecht zur Blendenklappe 22 verlaufenden Steg 30, der von der Röntgenstrahlung nicht getroffen wird. Durch eine Öffnung in der seitlichen Tubuswand 29 und in dem Steg 30 hindurch wirkt eine in einen Stellhebel 28 eingelassene Schraube 32 mit einem Fixierstück 31 zusammen, das mit einem Gewinde für die Schraube versehen ist. Durch Festziehen der Schraube 32 kann daher der Stellhebel 28 mit der Blendenklappe 22 verbunden werden, so daß diese mittels des Stellhebels schwenkbar ist. Durch dieses Festziehen werden der Steg und die Tubuswand elastisch federnd miteinander verspannt, so daß sich soviel Reibung ergibt, daß die Blendenklappe unter dem Einfluß der Schwerkraft ihre Stellung nicht ändert. Die für die Begrenzung des Strahlenbündels 25 wirksame hintere Ecke der oberen Blendenkante 23 fällt dabei mit der durch das Zentrum der Schraube 32 verlaufenden Schwenkachse 14 zusammen. Gegebenenfalls ist die Öffnung in dem Steg 30 so groß, daß diese Bedingung durch Verschiebung des Steges 30 vor dem Festziehen der Schraube 32 stets erfüllt werden kann.

In den Figuren 4 bis 6 sind verschiedene Ausführungen der Blendenklappe 22 dargestellt. Fig. 4 zeigt eine Blendenklappe, die - wie auch bei Fig. 2 und 3 - aus einer ebenen Platte besteht. Der untere Rand 15 dieser Platte, der die Ausblendung des Strahlenbündels 26 bestimmt, verläuft dabei parallel zu dem oberen Rand 23. Dadurch ergibt sich auf dem als Bildaufnehmer 4 dienenden Film an seinem hinteren Rand ein schmaler Aufbelichtungsstreifen 16 und an seinem vorderen Rand ein Strahlenfeld 17, dessen Breite von der Schwenkstellung der Blendenklappe 22 bestimmt wird. Nachteilig bei dieser Ausführungsform ist, daß das Strahlenfeld 17 stets rechteckig ist und somit der anatomischen Form der aufzunehmenden Mamma auch nicht annähernd angepaßt ist.

In dieser Hinsicht ist die Blendenklappe in Fig. 5 günstiger, die in einer zur Schwenkachse senkrechten Ebene einen L-förmigen Querschnitt aufweist, wobei der untere abgewinkelte Teil einen konkav geformten Rand 15 aufweist, der zumindest annähernd der Form der Mamma entspricht. Nachteilig an dieser Ausführungsform ist, daß auch in der Schwenkstellung der Blendenklappe 22, in der das Strahlenfeld 17 sein Maximum aufweist, stets ein Bereich an den beiden seitlichen Rändern des Filmformates abgeschirmt wird.

Die in Fig. 6 dargestellte Ausführungsform der Blendenklappe vermeidet die in Verbindung mit den Figuren 4 und 5 geschilderten Nachteile. Sie besteht aus einer ebenen Platte, jedoch ist deren unterer Rand konkav gekrümmt. Die Projektion dieses konkav gekrümmten Randes 15 auf den Film 4 ergibt ebenfalls eine gekrümmte Berandung des Strahlenfeldes 17. Der Krümmungsradius dieser Projektion ist um so kleiner, je kleiner die Fläche des Strahlenfeldes 17 ist. In der Extremstellung der Blendenplatte, in der sie mit dem Brennfleck der Strahlenquelle in einer Ebene liegt, geht die Projektion des gekrümmten Randes in eine Gerade über, wobei zwischen dem Streifen 16 und dem Strahlenfeld 17 nur ein schmaler durch die Dicke der Blendenplatte 22 vorgegebener Spalt verbleibt.

## Patentansprüche

1. Blendenanordnung für ein Mammographie-Röntgengerät zur Ausblendung eines Strahlenfeldes einer Strahlenquelle mit einem mit der Strahlenquelle verbundenen Tubus, der das Strahlenbündel entsprechend den äußeren Abmessungen eines Röntgenbildaufnehmers begrenzt, und einer darin befindlichen Blende, die das Strahlenbündel weiter eingrenzt, dadurch gekennzeichnet, daß die Blende durch eine Blendenklappe (22) aus einem die Röntgenstrahlung absorbierenden Material gebildet wird, die im Tubus (13) um eine zum vorderen Rand des Bildaufnehmers parallele Achse (14) schwenkbar angeordnet ist.

2. Blendenanordnung nach Anspruch 1, dadurch gekennzeichnet, daß eine Kantenecke (23) der Blendenklappe (22) mit der Schwenkachse (14) zusammenfällt und daß die Schwenkachse (14) im Strahlengang angeordnet ist.

3. Blendenanordnung nach einem der vorhergehenden Ansprüche, dadurch gekennzeichnet, daß die Blendenklappe (22) eben ist (Fig. 4, Fig. 6).

4. Blendenanordnung nach einem der Ansprüche 1 oder 2, dadurch gekennzeichnet, daß die Blendenklappe (22) in einer zur Schwenkachse senkrechten Ebene einen etwa L-förmigen Querschnitt aufweist (Fig. 5).

5. Blendenanordnung nach einem der vorhergehenden Ansprüche, dadurch gekennzeichnet, daß der die Ausblendung des Strahlenfeldes bestimmende Rand (15) der Blendenklappe (22) parallel zu ihrer Schwenkachse (14) verläuft.

6. Blendenanordnung nach einem der Ansprüche 1 bis 4, dadurch gekennzeichnet, daß der die Ausblendung bestimmende Rand (15) der Blendenklappe (22) konkav geformt ist.

## Claims

1. A diaphragm device for an X-ray mammographic apparatus for defining a radiation field of a radiation source, including a cone which is connected to the radiation source and which limits the radiation beam in accordance with the external dimensions of an X-ray image pick-up means, and also including a diaphragm which is situated therein and which further limits the radiation beam, characterized in that the diaphragm is formed by a diaphragm plate (22) which is made of an X-ray absorbing material and which is arranged in the cone (13) so as to be pivotable about an axis (14) which extends parallel to the front edge of the image pick-up means.

2. A diaphragm device as claimed in claim 1, characterized in that one edge corner (23) of the diaphragm plate (22) coincides with the pivot axis (14) which extends in the beam path.

3. A diaphragm device as claimed in any one of the preceding claims, characterized in that the diaphragm plate (22) is flat (Figures 4, 6).

4. A diaphragm device as claimed in one of the claims or 2, characterized in that the diaphragm plate (22) has an approximately L-shaped cross-section in a plane perpendicular to the pivot axis (Figure 5).

5. A diaphragm device as claimed in any one of the preceding claims, characterized in that the edge (15) of the diaphragm plate (22) which defines the radiation field extends parallel to the pivot axis (14) thereof.

6. A diaphragm device as claimed in any one of the claims 1 to 4, characterized in that the edge (15) of the diaphragm plate (22) which defines the radiation field is shaped so as to be concave.

## Revendications

1. Dispositif à diaphragme pour un appareil de mammographie par rayons X, destiné à diaphragmer un champ de rayonnement d'une source de rayonnement comportant un tube relié à la source de rayonnement, qui confine le faisceau de rayons d'une manière correspondant aux dimensions extérieures d'un dispositif radiographique, et un diaphragme logé dans ce tube qui limite davantage le faisceau de rayons, caractérisé en ce que le diaphragme est formé par un volet (22) en une matière absorbant les rayons X, qui est monté pivotant dans le tube (13) autour d'un axe (14) parallèle au bord antérieur du dispositif radiographique.

2. Dispositif à diaphragme suivant la revendication 1, caractérisé en ce qu'une arête (23) du bord du volet formant diaphragme (22) coïncide avec l'axe de pivotement (14) et que l'axe de pivotement (14) est disposé dans le trajet des rayons.

3. Dispositif à diaphragme suivant l'une quelconque des revendications précédentes, caractérisé en ce que le volet formant diaphragme (22) est plat (Fig. 4, Fig. 6).

4. Dispositif à diaphragme suivant la revendication 1 ou 2, caractérisé en ce que le volet formant diaphragme (22) présente, dans un plan perpendiculaire à son axe de pivotement, une section transversale en substance en L (Fig. 5).

5. Dispositif à diaphragme suivant l'une quelconque des revendications précédentes, caractérisé en ce que le bord (15) du volet formant diaphragme (22), qui est déterminant pour diaphragmer le champ de rayonnement, s'étend parallèlement à son axe de pivotement (14).

6. Dispositif à diaphragme suivant l'une quelconque des revendications 1 à 4, caractérisé en ce que le bord (15) du volet formant diaphragme (22), qui est déterminant pour le diaphragme, est de forme concave.

**FIG.1**

**FIG.2**

**FIG.3**

**FIG.4**

**FIG.5**

**FIG.6**